# EUROPEAN PATENT SPECIFICATION

(11) **EP 0 346 846 B1**
(45) Date of publication and mention of the grant of the patent: **01.09.1993**
(21) Application number: 89110715.3
(22) Date of filing: 13.06.1989
(51) Int. Cl.: A61B 3/10

(54) **Eye movement inspection device**
Augenbewegungsinspektionsgerät
Appareil pour l'inspection du mouvement des yeux

(30) Priority: 13.06.1988 JP 145425/88
(43) Date of publication of application: 20.12.1989
(73) Proprietor: KONAN CAMERA RESEARCH INSTITUTE INC., Nishinomiya-shi Hyogo (JP)
(72) Inventor: Kasahara, Tatsuya, Amagasaki-shi Hyogo (JP); Kishi, Kyuichi, Kobe-shi Hyogo (JP)
(74) Representative: Glawe, Delfs, Moll & Partner

(56) References cited:
- FR-A- 2 382 056
- US-A- 4 702 575
- SOVIET INVENTIONS ILLUSTRATED week 8634, 5 September 1986, p 31, abstract no. 223906/34, Derwent Publications Ltd., London, GB; & SU A 1204180.

## Description

### INDUSTRIAL FIELD OF APPLICATION

The present invention relates to optical vision devices employed in medical diagnosis, especially to a testing device related to observation, recording, and analysis of patient eyes by detecting vertical, horizontal, and rotational eye movement.

### PRIOR ART

Observation of patient eye movement is an indispensable diagnostic means for clinical investigation of inner-ear, balance, and central nerve dysfunction. Dizziness and these disorders have a recognized relationship, and by eye movement testing of patients the symptoms of the disorder can be known. Thus, a variety of eye movement detection devices have been employed up until now. The standard testing devices include the so-called ENG and EOG which employ the characteristic difference in electrical potential between the cornea and retina. The electrical potential difference of the eye movement is recorded on a polygraph device by placing electrodes on the skin surface near the eye sockets. The ENG extracts alternating current output for eye movement velocity, and the EOG extracts direct current output for eye movement fluctuations. A device called the PENG radiates infrared light on the left and right of the eyeball, extracts the intensity of reflected light of the cornea and sclera, and records eye movement on a polygraph. In addition, private practitioners mainly use a Fränzel lens device as a testing device to directly observe eye movement with a 20 diopter lens equipped with a light source. The recent devices are sold with an attached TV camera.

There is also a device called a search coil which mounts contact lenses with incorporated coils on the patient's eyes and extracts eye movement signals with a magnetic field device. More recently, a device with an infrared television camera that picks up reflected light from infrared radiation off of the eye and that extracts signals of eye movement from the camera has been disclosed in the literature (Ann Otol Rhinol Laryngol: 1977, Acta Otolarygol: 1987).

### PROBLEMS TO BE SOLVED BY THE INVENTION

Testing with the ENG and EOG devices incurs the affect of skin electrical potential noise, since the cornea-retina potential and skin potential are at the same level. The fluctuating potential that results generates deviations unless the vertical and horizontal leads from the skin electrodes are completely orthogonal; therefore, accurate detection proves to be problematic. Moreover, cycloduction movement of the eyes cannot be tested and testing requires a darkroom. The potentials of the cornea and retina remain unsteady and frequent corrections are required. The devices also receive the affects of blinking and eyelid movement, and when eyelids are closed to avoid visual fixation (involuntary viewing motion), vertical eye vibrations (eye movement in the vertical direction) occur. Consequently, true eye movement cannot be observed, and this problem is compounded by a separate target display that requires a large space.

Testing with the PENG device also incurs the affects of blinking or eyelid movement and must take place with open eyes. Since the eyes are fixed, nystagmus is diminished. Although there is no affect from skin potential, testing proceeds in a darkroom in the same way as ENG and EOG, while a separate target display is still required. Finally, cycloduction movement cannot be tested. The devices based on the Fränzel lens allow visual confirmation of cycloduction and testing can proceed in a lighted room. But eyes still do not follow the displayed target and visual fixation cannot be completely eliminated. Besides these disadvantages, eye movement reaction cannot be continuously recorded (hence analyzed) for long periods. The search coil devices require the mounting of contact lenses on the patient and testing must proceed inside a magnetic-field device. Hence, patient freedom is minimal, and eye movement cannot be observed close to a natural condition. In addition, all devices employing reflected light from infrared radiation and cited in the literature noted above require darkrooms, and the procedures for testing become complicated.

An eye movement monitoring or testing device according to the preamble of claim 1 is disclosed in US-A-4 702 575. This device comprises a head mounting provision in which an infrared radiating means is established to irradiate the eye of a patient to be examined, and an infrared light detector which measures the movement of the cornea of the eye. In this device a virtual point image is formed behind a surface of the cornea, and the incident light is parallel light and the cornea serves as a converging lens. The infrared light detector detecs displacements of the virtual point image.

An eye position detector employing a television camera is disclosed in FR-A-2 382 056.

Soviet inventions illustrated, week 8634, 5 September 1986, page 31, abstract no. 223906/34, Derwent Publications Ltd., London, GB, describes an arrangement of an eye movement recording system in which one channel separates the horizontal component of eye motion and the other channel the vertical component.

It is therefore an object of the invention to provide an eye movement monitoring or testing device without any skin potential effects whatsoever, with large patient freedom during testing without restricting patient head movement, without visual fixation and with accurate detection of vertical, horizontal and rotational eye movement in a lithted room, whereby observation, recording and analysis can be performed.

It is a further object of the invention to provide an eye movement monitoring or testing device eleminating the requirements of a wide space for target display and wherein simultaneous observation of both eyes is allowed, and wherein accurate adjustments are allowed according to the eye conditions of the patient such as eye width etc.

These objects are archievid by an eye movement monitoring device as defined in claim 1; the dependent claims are related to furthere developments of the invention.

According to the invention a dark room is formed on the face of the patient by means of a goggles or mask type housing having a flexible seal or shutter piece contacting the face and head of the patient to shut out visible light entering the patient's eyes, wherein the goggles are equipped with a provision to mount them on the patient's head and move together. Inside said goggles, an infrared radiating piece is established for irradiating the patient's eyes, and an infrared detecting television camera is mounted facing the eyes of the patient so the optical axis faces the patient and the infrared image of the patient's eye movement can be sensed, whereby the image signals are extracted from said camera for testing. Inside the goggles described above, it is preferable to establish reflection plates that reflect and disperse the infrared light that illuminates the patient's eyes and targets which are visible-light source to irradiate the prescribed visible light on the eyes.

In addition, to correspond to the left and right eyes of the patient, the infrared radiating piece, infrared detecting television camera, reflection plates, and targets can be established as a unit, wherein two units can form a pair of mobile eyepieces. The television camera optical axes of the respective mobile eyepieces are established for effective use with an optical axis adjustment device for adjustable movement horizontally, which is the direction of eye width, vertically, and the direction of the ocular axis. An infrared LED is suitable for the above infrared radiation piece, and as targets multiple visible-light emitting diodes (LEDs) with display switching enabled should be preferably established on a spherical surface centered as the center of eye rotation. Said targets are established in a cross shape vertically and horizontally from the intersection of targets on the ocular axes, hence on a reflection plate with a prescribed shape, the reflection surfaces being spherical surfaces, and wherein the optical axis of the television camera is positioned in proximity to said intersection targets and projecting through the reflection plate. For the head-mounting provision of the goggles, air bands are efficient.

### ACTION

When the eye movement detection device arranged as described above is mounted on a patient's head, the shutter piece of the goggles forms a darkroom in front of the patient's eyes inside the goggles, and the infrared light from the infrared radiating piece such as an infrared LED is reflected and dispersed by the reflection plates irradiating the eyes, whereupon the image signals of the patient's eye movements are extracted from the infrared detecting television camera. Because of the darkroom formation in front of the eyes, visual fixation is eliminated, and because the goggles move together with the patient's head, patient freedom is enhanced. Consequently, testing of free eye movement is enabled, and observation, recording, and analysis of eye movement including cycloduction is permitted from the image signals.

In addition to eliminating the darkroom, by establishing targets consisting of visible-light sources inside, the goggles also eliminate the wide space required for establishing targets. By positioning multiple targets on a spherical surface centered around the center of eye rotation, accurate observation of eye movement without target position correction is permitted.

Moreover, the infrared radiating piece, infrared detecting television camera, reflection plates, and targets can be established as a unit, whereby two units form a pair of mobile eyepieces to facilitate simultaneous observation of both eyes. The respective mobile eyepieces are established to enable accurate adjustment in response to individual differences with an optical axis adjustment device for adjustable movement vertically, horizontally, which is the direction of eye width, and the direction of ocular axis, thereby allowing accurate testing of eye movement. Finally, an air band simplifies the mounting operation of goggles on the patient's head.

### EMBODIMENT

The following preferred embodiment is described along with the attached drawings.

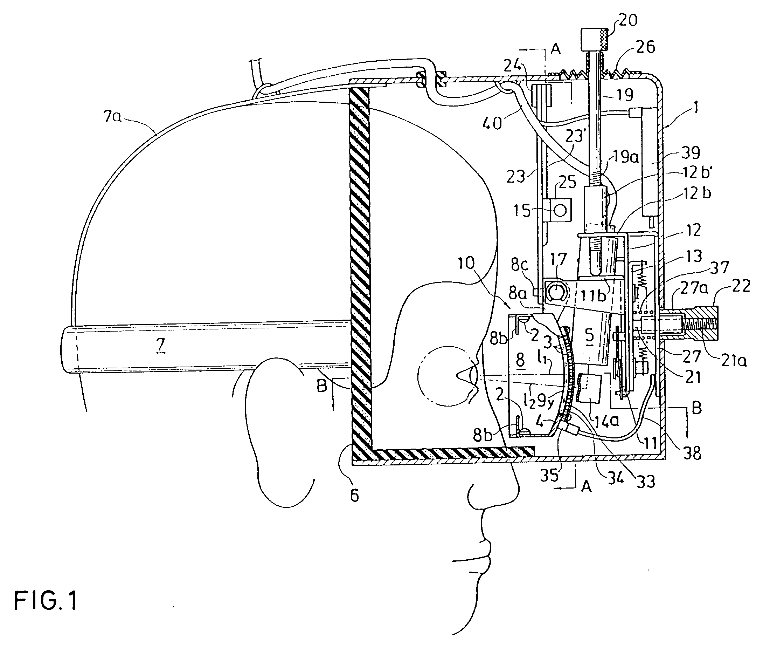

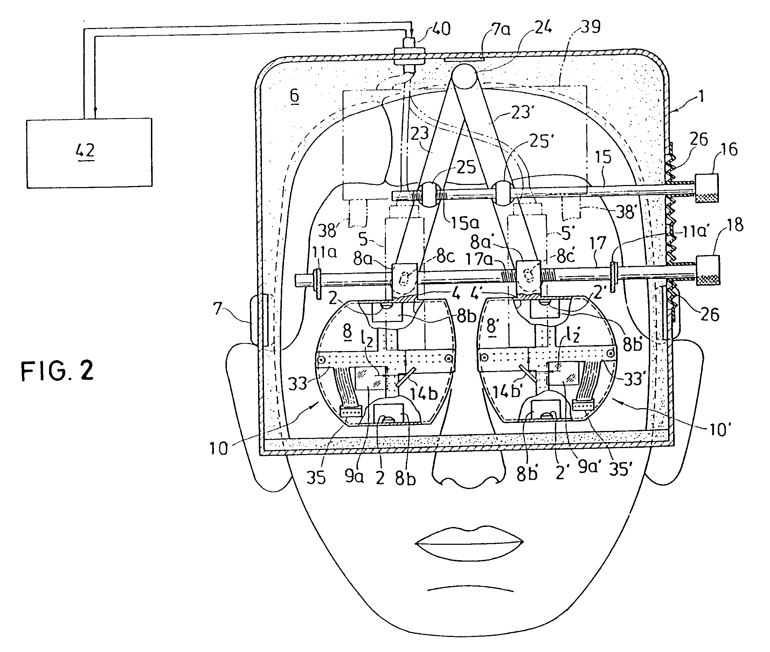

Figure 1 is a side view showing the eye movement testing device of the present invention mounted on a patient with crucial areas shown as a cross section, and Figure 2 is a front view of Figure 1, essentially a cross section along the line A-A.

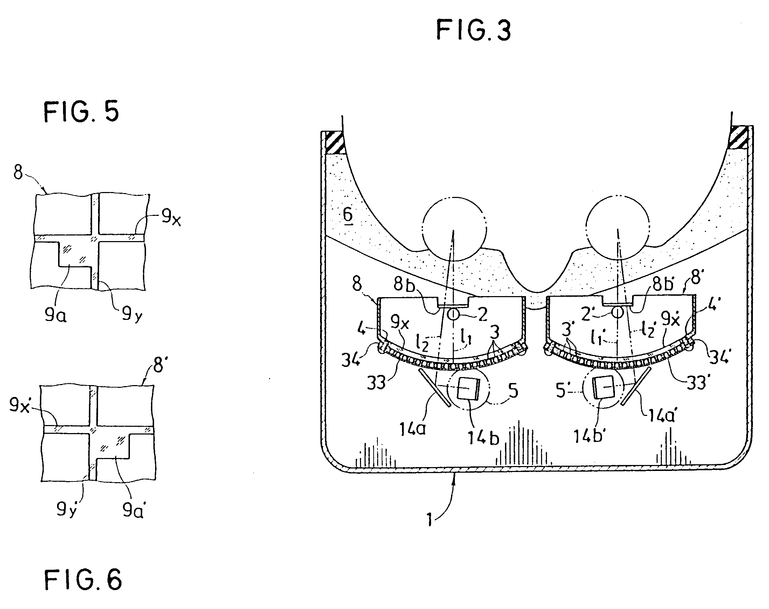

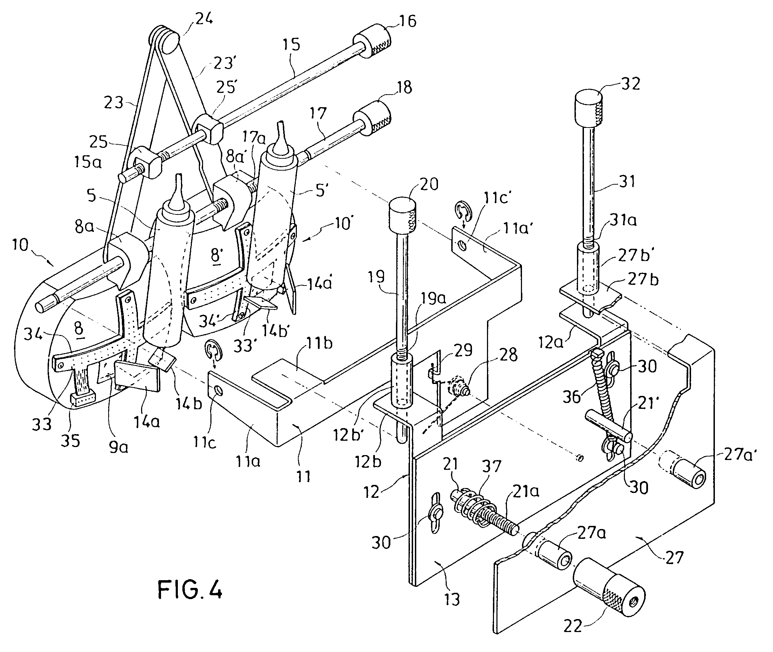

The goggles (1) are made from light alloy or polyester with glass fiber, are mounted on the patient's face to form a darkroom in front of both eyes of the patient, and are formed in a prescribed box shape to store therein a pair of eyecap-shaped mobile eyepieces (10), (10′) for eye movement observation described below. Along the joining area to the patient's face and head, a shutter piece (6) consisting of flexible shutter material such as urethane foam is established over the goggles to match with the folds of the patient's face. The left and right sides of the goggles (1) contacting the patient's face blow up by injection of air to a prescribed pressure and tighten on the head, whereupon both ends of the hollow-interior, rubber air band (7) which fastens the goggles on the patient are fixed and the air-band middle at the back of the patient's head hangs from a band (7a) from the top of the goggles. This air band (7) expands under pressure of compressed air sent from an air pump, not illustrated, according to operation by an operator and fixes to the patient's head, and also is easily removed by reducing the pressure and shrinking. This pressure is always a constant pressure; therefore, the differences in mounting and fastening for individuals can be diminished. Corresponding to both left and right eyes of the patient, a pair of mobile eyepiece parts (10), (10′) that detect the infrared images of the eyes upon infrared radiation are established within the goggles (1) in a floating condition, adjustable in mutual spacing, the X direction (horizontal), Y direction (vertical), and Z direction (ocular axis direction). Said mobile eyepiece parts (10), (10′) are equipped with eyecaps (8), (8′) that also function as reflection plates, and said eyecaps (8), (8′) incorporate parts with infrared LEDs (IR-LEDs) (2), (2′) that radiate the eyes of the patient with infrared, with reflection plates (4), (4′) that reflect and disperse the infrared light from said diodes in order to illuminate the eyes of the patient, and with a group of targets (3), (3′) consisting of LEDs to radiate prescribed visible light on the patient's eyes. In addition, ultra-miniature infrared detecting television cameras (5), (5′) equipped with CCDs for infrared image detection of the patient's eyes are mounted together with two optical-axis deflecting reflection mirrors. The eyecaps (8), (8′) consist of an inner box centered at essentially the rotating center of the eyes which is formed in the prescribed box shape equipped with reflection plates (4), (4′) having spherical reflection surfaces. On the front, inner surfaces of the flat, upper and lower walls, infrared LEDs (IR-LEDs) are located in front of the vertical walls (2), (2′) established on said upper and lower walls between both ocular-axis directions (the wiring is abridged on the drawings). The reflection plates (4), (4′) have prescribed coatings on the reflecting surfaces, and multiple LEDs (3), (3′) form a target group in a cross-shaped pattern which intersects at a target LED located essentially on the ocular axes (11), (11′) of reflection plate (4), (4′) centers and are positioned on a flexible substrate (33), (33′) via support pieces (34), (34′) behind the cross-shaped transparent windows (9x), (9y) and (9x′), (9y′) (see Figure 1, Figure 3, and Figure 5). In proximity to the cross-shaped target intersection, prescribed, rectangular, transparent window (9a), (9a') sections are established in the area of the eyes' infrared-image optical paths next to the aforesaid cross-shaped transparent window portions, actually the transparent windows in the X direction (horizontal) (9x), (9x') and the transparent windows in the Y direction (vertical) (9y), (9y'), to the lower left of the intersection for the left eyecap (8) and to the lower right of the intersection for the right eyecap (8') (left and right are herein defined as the view from the operating side when the goggles are mounted) (see Figure 5 and Figure 6). Behind both rectangular, transparent window sections (9a), (9a'), horizontal deflection mirrors (14a), (14a') are established so that the optical paths of the eye infrared-images from each eye face the pair of infrared detecting television cameras (5), (5'), positioned vertically upward and slightly inward from the left and right ocular axes, hence so that the optical axes (ℓ₂), (ℓ₂') of said infrared television cameras (5), (5') face each eye. The optical paths thus deflected allow light to enter the infrared detecting television cameras (5), (5′) via the mirrors (14b), (14b′) that deflect the light vertically upward (see Figure 2, Figure 3, and Figure 4). Said television cameras (5), (5′) employ fixed-focus lenses. In this way, the mobile eyepiece parts (10), (10′) can have a compact configuration. The LEDs (3), (3′) mounted on the aforesaid flexible substrates (33), (33′) light via the connector terminals (35), (35′) mounted on the back of the eyecaps, and their control proceeds by joining the connector-terminal leads of flexible cable (38), (38′) to the aforesaid terminals (35), (35′) and via the target controller (39) established on the inside of the goggles (1) and operated by control signals from outside the goggles.

The aforesaid pair of eyecaps (8), (8′) has respective unified guide blocks (8a), (8a′) above equipped with guide holes that regulate the left and right movement of the eyecaps, wherein the guide blocks (8a), (8a′) permit horizontal position adjustment of the right eyecap (8′) position by rotation of a horizontal movement knob (18) that is exposed on the outside of the goggles (1), where above a horizontal movement shaft (17) equipped with the horizontal movement knob (18), the left guide block (8a) is fitted to said movement shaft (17) and positioned to freely swing and the right guide block (8a′) is threaded to the male threading (17a) formed on said movement shaft (17). Both guide blocks (8a), (8a′), moreover, have pins (8c), (8c′) imbedded in the front (the patient's side), and the pins (8c), (8c′) are joined to freely swing through long holes formed on the lower ends of a pair of arms (23), (23′) pivot-mounted to rotate freely on their upper ends from an arm shaft (24). Near the centers of the aforesaid arms (23), (23′), a pair of shaft supporting blocks (25), (25′) are attached to the arms (23), (23′) to rotate freely, supporting the eye-width adjustment shaft (15) which matches the eye width of the patient and changes the spacing of the pair of arms (23), (23′), in other words the mutual space between the pair of eyecaps (8), (8′). The side (the left side) of one supporting block threads together with the male threading (15a) formed on the eye-width adjustment shaft, and the other side (right side) fits to said eye-width adjustment shaft (15) while allowing rotation and a fastened condition for the left and right position relationship of said shaft (15). By rotating the eye-width adjustment knob (16) projecting outside the goggles (1), the mutual space changes between the eyecap pair. Hence, by turning the eye-width adjustment knob (16), the space between the pair of eyecaps (8), (8′), which is the opening of the pair of arms (23), (23′), or eye width, can be adjusted.

The aforesaid horizontal adjustment shaft (17) is supported by a rotation frame (11) established to allow rotation centered around the longitudinal axis (Z-axis), wherein the horizontal slope, which is the slope of the eye-width direction, can be changed. Said shaft (17) is supported by a rotation-frame support shaft (28) (see Figure 4) for free rotation imbedded in the prescribed position of the elevation frame (12) which is located central to the multiple mobile frames (11), (12), (13). Said shaft (17) is thus supported by the support arms (11a), (11a′) on both left and right sides of the rotation frame (11) braced clockwise by a rotating spring (29), with said shaft (17) inserted through holes (11c), (11c′) punched on the arms (11a), (11a′), and stopped by a pair of E rings. By rotating the eye-width direction slope adjustment shaft (19) with male threading (19a) threaded to a bushing (12b′) established on the upper left, bent part (12b) of the elevation frame (12) using the eye-width direction slope adjustment knob (20) exposed to the outside of the goggles (1), the upper left, bent part (11b) of the rotation frame (11) is driven by the lower end of said adjustment shaft (19) to allow eye-width direction slope adjustment. The aforesaid elevation frame (12) is bolstered upward by tension springs (36) supported and guided by three guide pins (30) on the longitudinal movement frame (13). At the upper right, bent part (12a) of the elevation frame (12), the vertical position of the elevation frame (12) can be adjusted by rotating the vertical adjustment shaft (31) with the vertical adjustment knob (32) projecting outside the goggles (1) and by contacting the leading end of said shaft (31) threaded together with the male threading (31a) of the bushing (27b′) attached to the upper right, bent part (27b) of the support plate fastened on the inside of the back wall of the goggles (1). The aforesaid longitudinal movement frame (13) can swing forward and backward with fitted insertions of a pair of longitudinal movement shafts (21), (21′) imbedded in said longitudinal movement frame to the pair of guide bushings (27a), (27a′) attached to the aforesaid support plate (27). Between said pair of longitudinal movement shafts (21), (21′), the long, longitudinal movement shaft (21) on the left has a prescribed, long, male-threaded section (21a) formed from the end, and the male-threaded section (21a) with a left guide bushing inserted and projecting therethrough threads together with a longitudinal adjustment knob (22) with a female-threaded section. The front end surface of said knob (22) contacts the support plate (27) by a compression spring (37) fit into the longitudinal movement shaft (21), and the aforesaid longitudinal movement frame (13) moves forward and backward by turning said knob.

In the sections where the aforesaid eye-width adjustment shaft (15), horizontal adjustment shaft (17), eye-width directional slope adjustment shaft (19), and vertical adjustment shaft (31) project through the outer wall of the goggles, multi-ringed, corrugated, flexible light-shutter plates (26) fit around these adjustment shafts and provide complete shut-out of light, even when adjusting positions with the pair of mobile eyepiece parts (10), (10′) and the aforesaid adjustment shafts (15), (17), (19), (31) move, permitting rotation for the knobs (16), (18), (20), (32) located outside said shutter plate (26) and following the movement.

To test patient eye movement employing the testing device of the aforesaid embodiment, first the patient's eye width is measured with a scale. This dimension is set between the target center of the target groups of the pair of mobile eyepiece parts (10), (10′) within the goggles (1) before the patient mounts them. Next, the goggles (1) are placed on the patient's head with no air feeding to the the air band (7), and both mobile eyepiece parts are faced toward both eyes. Then air is sent to the the air band (7) from an air pump by personal computer operation of an operator. The air band becomes pressurized by compressed air and fastens the goggles to the patient. Next, via cables (40) prescribed power is supplied to the infrared detecting television cameras (5), (5′) and infrared LEDs (IR-LEDs) in each mobile eyepiece part (10), (10′) of the goggles. The infrared LEDs (2), (2′) emit radiation which is reflected and dispersed from the vertical plates (8b), (8b′) of the eyecaps (8), (8′) by the reflection plates (4), (4′). The light from the patient's eyes evenly illuminated with infrared light passes the cross-shaped transparent windows (9a), (9a′) and enters the infrared detecting television cameras (5), (5′) as two mirrors deflect its optical paths. The image signals from said television cameras (5), (5′) are sent to the analyzer (42) via cabling (40), and signals from said analyzer (42) display images on a monitor that is not illustrated.

Position adjustment of the mobile eyepiece parts (10), (10′) proceeds by viewing this image. At this time, position adjustment for the right mobile eyepiece part (10′) in X, Y, and Z directions proceeds by employing the various adjustment knobs (18), (26), (22) to display the left eye image of the patient at the prescribed location on the monitor. Next, the slope of the eye-width direction is adjusted with the knob (20) to correctly display both eyes. After this, corrections for individual differences in the patient's eyes are performed. Optical stimuli provided by lighting the LED targets (3), (3′) according to the prescribed testing by moving such lighted points horizontally and vertically via the target controller (39) in the goggles (1) with the control signals emitted by the analyzer (42) demonstrates eye-movement conditions of the patient, such as viewing of moving targets, which enter the two infrared detecting television cameras (5), (5′) as infrared images. Image signals from said television cameras (5), (5′), sync-driven by sync signals from the analyzer (42), are extracted and processed as desired when sent to the analyzer's image processor, and displayed on the external monitor for visual inspection in addition to the prescribed test recording on a prescribed recording device.

The testing device of the present invention is not restricted by the preferred embodiment stated above, but is freely modifiable into a variety of configurations that do not diverge from the concept of the present invention as claimed.

### EFFECT

As clear from the above description, the present invention provides the following effects.

In the eye movement testing device of claim 1, the shutter piece attached to the goggles matches the folds of the face and completely covers the gaps between the goggles, thereby forming a darkroom in front of the patient's eyes. The patient reaches darkness visually, and since the goggles move together with the patient's head, the entire testing room need not be made into a dark room, the visual fixation of the patient is eliminated, and the movements of the patient are not limited. Thus, with the infrared radiation piece established to radiate the patient's eyes in the goggles and with optical axes of the infrared detecting television cameras positioned to face the patient's eyes, observation, recording, and measurement of free eye movement including cycloduction movement in a lighted room can be performed when extracting eye movement image signals. The infrared illumination enables lighting of eyes in a dark condition without subjecting the patient to a bright glare. In the eye movement testing device of claim 2, the effective illumination of the patient's eyes by reflection plates for reflection and dispersion of infrared light help image processing of the image signals from the infrared detecting television cameras.

In the eye movement testing device of claim 3, the targets which are visible light sources are equipped within the goggles and a large space for establishing targets is not required.

In the eye movement testing device of claim 4, an infrared radiation device, infrared detecting television camera, targets, and reflection plates are all established as pairs inside the goggles, corresponding to both left and right eyes; therefore, simultaneous observation of both eyes is possible.

In the eye movement testing device of claim 5, when the goggles are mounted on the patient, the pair of mobile eyepiece parts each incorporating an infrared radiation piece, infrared detecting television camera, targets, and reflection plates can be adjusted horizontally, vertically, and in the ocular-axis direction corresponding to the patient's eyes with the optical-axis adjustment device.

Thus, ocular axes and camera optical axes can be accurately adjusted regardless of the patient, and comprehensive visual observation and accurate testing results are obtained on the monitor. In addition, since the targets and television cameras maintain a constant position relationship, adjustments and observation data corrections are simple.

Finally, the ocular-axis direction can be adjusted, permitting a constant distance between the mobile eyepiece parts and the eyes, and therefore eliminating visual angle correction for targets.

In the eye movement testing device of claim 6, the fact that the infrared radiation piece is an infrared LED permits establishment in the goggles and easy control.

In the eye movement testing device of claim 7, since the targets are multiple LEDs emitting visible light located on a spherical surface centered on the center of eye rotation with display switching enabled, their establishment and control is simple. Furthermore, the target positions require no correction for accurate eye movement observation.

In the eye movement testing device of claim 8, targets are established in a cross shape vertically and horizontally from the intersection of targets on the ocular axes, hence on a reflection plate with a prescribed shape, the reflection surfaces being spherical surfaces, and wherein the optical axis of the television camera is positioned in proximity to said intersection targets and projecting through the reflection plate. Thus, the light points which become targets can be moved in horizontal and vertical directions to the advantage of eye movement testing by giving optical stimuli to the patient. Moreover, the position relationship between the targets and television cameras can be placed advantageously for testing.

In the eye movement testing device of claim 9, since the head-mounting provision of the goggles is an air band, mounting and dismounting for the patient is easy. Because pressurization is always constant, the differences in mounting for individuals can be decreased.

### 4. BRIEF DESCRIPTION OF DRAWINGS

The drawings demonstrate an embodiment of the present invention.
Figure 1 is a side view showing the eye movement testing device of the present invention mounted on a patient with crucial areas shown as a cross section,
Figure 2 is a front view of Figure 1, essentially a cross section along the line A-A,
Figure 3 is a cross section showing the relationship between the eyecaps and eyes in the goggles along the line B-B,
Figure 4 is a diagonal view of the configuration of the mobile eyepiece part drive mechanism which is the optical-axis adjustment device of the infrared detecting television camera,
Figure 5 is a partial back view showing the central transparent window section of the right eye's eyecap, and
Figure 6 is a partial back view showing the central transparent window section of the left eye's eyecap.

(1) ... goggles, (2), (2′) ... infrared radiation piece (infrared LED), (3), (3′) ... target (LED), (4), (4′) ... reflection plate, (5), (5′) ... infrared detecting television camera, (6) ... shutter piece, (7) ... head mounting provision (air band), (8), (8′) ... eyecap, (9x), (9y), (9a) ... transparent window, (10) ... mobile eyepiece part, (11) ... rotation frame, (12) ... elevation frame, (13) ... longitudinal movement frame, (15) ... eye-width adjustment shaft, (16) ... eye-width adjustment knob, (17) ... horizontal movement shaft, (18) ... horizontal movement knob, (19) ... eye-width direction slope adjustment shaft, (20) ... eye-width directional slope adjustment knob, (22) ... longitudinal adjustment knob.

## Claims

1. Eye movement monitoring device comprising a head-mounting provision (7), wherein an infrared radiating means (2,2') is established to irradiate the eye of a patient and a detecting means (5,5') for detecting infrared light is mounted so that its optical axis faces towards an eye of the patient, whereby said detecting means outputs signals of eye movements of the patient,
**characterized** in that
said monitoring device comprises a mask or goggles (1,) provided with a seal or shutter piece (6) to flexibly contact the face and head of the patient for shutting out visible light entry, whereby forming a dark room in front of the eyes of the patient,
said infrared radiating means (2,2') is adapted to uniformly irradiate the eye of the patient, and
that said detecting means (5,5') is a television camera obtaining the whole image of the eye and outputting image signals.

2. An eye movement monitoring device as claimed in claim 1, wherein reflection plates (4,4') are arranged in the goggles (1,) so as to reflect infrared light in various directions and disperse it in order to illuminate the eye of the patient uniformly.

3. An eye movement monitoring device as claimed in claims 1 or 2, comprising targets (3,3') comprised of visible light sources for irradiating visible light on the eyes of the patient.

4. An eye movement monitoring device as claimed in claim 3, wherein said infrared radiating means (2,2'), infrared detecting television camera (5,5') for detecting both the horizontal and vertical movements and the cycloduction of the eye, reflection plates (4,4'), and targets (3,3') are all established as pairs corresponding to the left and right eyes of the patient.

5. An eye movement monitoring device as claimed in claim 4, wherein a pair of mobile eyepiece parts (10,10') corresponding to the left and right eyes of the patient are each formed by the infrared radiating means (2,2'), the infrared detecting television camera (5,5'), the reflection plates (4,4'), and targets (3,3') as a unit, and wherein an optical-axis adjustment device movably supports and adjusts said mobile eyepiece parts (10,10') in three directions including a horizontal direction of the width of eyes, a vertical direction perpendicular to the horizontal direction and a direction of ocular axis of the eye so that the optical axes of said television cameras (5,5') face towards the eyes of the patient.

6. An eye movement monitoring device as claimed in claims 1 to 5, wherein the infrared radiating means (2,2') is an infrared light-emitting diode (LED).

7. An eye movement monitoring device as claimed in claims 3 to 6, wherein the targets (3,3') consist of a plurality of visible-light emitting diodes (LEDs) which are located on a spherical surface centered at the center of eye rotation, each visible-light emitting diode being switchable on and off so that the patient looks at a visible light emitting diode being turned on.

8. An eye movement monitoring device as claimed in claim 7, wherein the targets (3,3') are established in a cross shape vertically and horizontally from intersections of targets so that the intersections being on the ocular axes of the eyes and established on reflection plates (4,4') having spherical surfaces of a prescribed shape, and wherein said television cameras (5,5') are positioned so that their optical axis pass through the respective reflection plate (4,4') in proximity to said intersections.

9. An eye movement monitoring device as claimed in claims 1 to 8, wherein the head-mounting provision (7) for the goggles (1) is an air band.

## Patentansprüche

1. Augenbewegungs-Überwachungsgerät mit einer Vorrichtung (7) zur Befestigung am Kopf, wobei eine Infrarot-Strahlungseinrichtung (2, 2') vorgesehen ist, um das Auge eines Patienten zu bestrahlen, und eine Detektoreinrichtung (5, 5') zur Erfassung von Infrarotlicht so eingerichtet ist, daß ihre optische Achse auf das Auge des Patienten gerichtet ist, wobei die Detektoreinrichtung Signale entsprechend der Augenbewegungen des Patienten abgibt;
dadurch **gekennzeichnet,**
daß das Überwachungsgerät eine Maske oder Brille (1) aufweist, die mit einem Dichtungs- oder Schließstück (6) versehen ist, das sich schmiegsam an das Gesicht und den Kopf des Patienten anlegt, um den Eintritt von sichtbarem Licht auszuschließen, wodurch vor den Augen des Patienten ein Dunkelraum gebildet wird,
daß die Infrarot-Strahlungseinrichtung (2, 2') das Auge des Patienten gleichmäßig ausleuchtet, und
daß die Detektoreinrichtung (5, 5') eine Fernsehkamera ist, die das gesamte Bild des Auges erhält und Bildsignale abgibt.

2. Augenbewegungs-Überwachungsgerät nach Anspruch 1, wobei Reflexionsplatten (4, 4') in der Brille (1) vorgesehen sind, um Infrarotlicht in verschiedene Richtungen zu reflektieren und zu streuen, um das Auge des Patienten gleichmäßig auszuleuchten.

3. Augenbewegungs-Überwachungsgerät nach Anspruch 1 oder 2, mit Zielobjekten (3, 3'), die Quellen für sichtbares Licht aufweisen, um sichtbares Licht auf die Augen des Patienten zu richten.

4. Augenbewegungs-Überwachungsgerät nach Anspruch 3, wobei die Infrarot-Strahlungseinrichtung (2, 2'), die Infrarot-detektierende Fernsehkamera (5, 5') zum Detektieren sowohl der horizontalen als auch vertikalen Bewegungen und der Kreisführung des Auges, die Reflexionsplatten (4, 4') und die Zielobjekte (3, 3') jeweils paarweise entsprechend dem rechten und linken Auge der Patienten angeordnet sind.

5. Augenbewegungs-Überwachungsgerät nach Anspruch 4, bei dem ein Paar mobiler Okulare (10, 10') entsprechend dem linken und dem rechten Auge des Patienten jeweils als Baueinheit aus der Infrarot-Strahlungseinrichtung (2, 2'), der Infrarot-detektierenden Fernsehkamera (5, 5'), den Reflexionsplatten (4, 4') und den Zielobjekten (3, 3') gebildet sind und eine Einrichtung zum Einstellen der optischen Achse die mobilen Okulare bewegbar trägt und in drei Richtungen einstellt, eischließlich einer horizontalen Richtung des Augenabstandes, einer vertikalen Richtung senkrecht zur horizontalen Richtung und einer Richtung der Okularachse des Auges, so daß die optischen Achsen der Fernsehkameras (5, 5') auf die Augen des Patienten gerichtet sind.

6. Augenbewegungs-Überwachungsgerät nach Anspruch 1 bis 5, wobei die Infrarot-Strahlungseinrichtung (2, 2') eine Infrarotlicht emittierende Diode ist (LED).

7. Augenbewegungs-Überwachungsgerät nach Anspruch 3 bis 6, wobei die Zielobjekte (3, 3') aus einer Vielzahl sichtbares Licht emittierender Dioden (LEDs) bestehen, die auf einer sphärischen Fläche liegen, die zum Mittelpunkt der Augendrehung zentriert ist, wobei jede sichtbares Licht emittierende Diode ein- und ausschaltbar ist, so daß der Patient auf eine sichtbares Licht emittierende Diode schaut, die eingeschaltet wird.

8. Augenbewegungs-Überwachungsgerät nach Anspruch 7, wobei die Zielobjekte in Kreuzform vertikal und horizontal ausgehend von Kreuzstellen der Ziele ausgebildet sind, so daß die Kreuzungsstellen auf den Okularachsen der Augen liegen und auf Reflexionsplatten (4, 4') errichtet sind, die eine sphärische Fläche vorgeschriebener Form haben, und wobei die Fernsehkameras (5, 5') so positioniert sind, daß ihre optischen Achsen durch die jeweiligen Reflexionsplatten (4, 4') in der Nähe der Kreuzstellen verlaufen.

9. Augenbewegungs-Überwachungsgerät nach Anspruch 1 bis 8, wobei die Vorrichtung (7) der Brille (1) zum Befestigen am Kopf ein Luftband ist.

## Revendications

1. Dispositif de surveillance des mouvements des yeux comportant un moyen (7) de montage sur la tête, dans lequel un moyen (2, 2') irradiant des infrarouges est établi de façon à irradier l'oeil d'un patient, et un moyen de détection (5, 5') destiné à détecter une lumière infrarouge est monté de façon que son axe optique soit tourné vers un oeil du patient, afin que ledit moyen de détection délivre en sortie des signaux des mouvements des yeux du patient,
caractérisé en ce que
ledit dispositif de surveillance comporte un masque ou des lunettes (1) pourvus d'un joint ou d'une pièce d'obturation (6) destiné à être en contact souple avec le visage et la tête du patient pour s'opposer à l'entrée de la lumière visible, formant ainsi une chambre noire devant les yeux du patient,
ledit moyen (2, 2') irradiant des infrarouges est conçu pour irradier uniformément l'oeil du patient, et
ledit moyen de détection (5, 5') est une caméra de télévision obtenant l'image entière de l'oeil et délivrant en sortie des signaux d'image.

2. Dispositif de surveillance des mouvements des yeux selon la revendication 1, dans lequel des plaques réfléchissantes (4, 4') sont agencées dans les lunettes (1) de manière à réfléchir la lumière infrarouge dans diverses directions et à la disperser pour éclairer uniformément l'oeil du patient.

3. Dispositif de surveillance des mouvements des yeux selon la revendication 1 ou 2, comportant des cibles (3, 3') constituées de sources de lumière visible pour irradier de lumière visible les yeux du patient.

4. Dispositif de surveillance des mouvements des yeux selon la revendication 3, dans lequel ledit moyen (2, 2') irradiant des infrarouges, ladite caméra (5, 5') de télévision pour la détection des infrarouges destinée à détecter les mouvements à la fois horizontaux et verticaux et la cycloduction de l'oeil, lesdites plaques réfléchissantes (4, 4') et lesdites cibles (3, 3') sont tous établis par paires correspondant aux yeux gauche et droit du patient.

5. Dispositif de surveillance des mouvements des yeux selon la revendication 4, dans lequel deux oculaires mobiles (10, 10') correspondant aux yeux gauche et droit du patient sont formés chacun par le moyen (2, 2') irradiant des infrarouges, la caméra (5, 5') de télévision pour la détection des infrarouges, les plaques réfléchissantes (4, 4') et les cibles (3, 3'), d'un seul bloc, et dans lequel un dispositif de réglage de l'axe optique supporte de façon mobile et règle lesdits oculaires mobiles (10, 10') dans trois directions comprenant une direction horizontale de la largeur des yeux, une direction verticale perpendiculaire à la direction horizontale et une direction de l'axe oculaire de l'oeil afin que les axes optiques desdites caméras (5, 5') de télévision soient tournés vers les yeux du patient.

6. Dispositif de surveillance des mouvements des yeux selon les revendications 1 à 5, dans lequel le moyen (2, 2') irradiant des infrarouges est une diode électroluminescente (DEL) à infrarouges.

7. Dispositif de surveillance des mouvements des yeux selon les revendications 3 à 6, dans lequel les cibles (3, 3') sont constituées de plusieurs diodes électroluminescentes (DEL) à lumière visible qui sont placées sur une surface sphérique centrée au centre de rotation de l'oeil, chaque diode électroluminescente à lumière visible pouvant être allumée et éteinte afin que le patient regarde une diode électroluminescente à lumière visible qui est allumée.

8. Dispositif de surveillance des mouvements des yeux selon la revendication 7, dans lequel les cibles (3, 3') sont établies en forme de croix verticalement et horizontalement à partir d'intersections des cibles afin que les intersections se trouvent sur les axes oculaires des yeux et soient établies sur des plaques réfléchissantes (4, 4') ayant des surfaces sphériques d'une forme prédéterminée, et dans lequel lesdites caméras (5, 5') de télévision sont placées de façon que leurs axes optiques passent par les plaques réfléchissantes respectives (4, 4') à proximité desdites intersections.

9. Dispositif de surveillance des mouvements des yeux selon les revendications 1 à 8, dans lequel le moyen (7) de montage sur la tête pour les lunettes (1) est un bandeau pneumatique.
